Europäisches Patentamt

European Patent Office

Office européen des brevets

(11) Publication number: **0 354 818**
**A2**

## EUROPEAN PATENT APPLICATION

(21) Application number: 89308214.9

(22) Date of filing: 14.08.89

(51) Int. Cl.⁵: **A 61 K 31/135**
**A 61 K 31/655, A 61 K 31/19**

(30) Priority: 12.08.88 IL 87444

(43) Date of publication of application:
14.02.90 Bulletin 90/07

(84) Designated Contracting States:
AT BE CH DE ES FR GB GR IT LI LU NL SE

(71) Applicant: **HADASSAH MEDICAL ORGANIZATION**
**Kiryat Hadassah P.O. Box 12000**
**IL-91120 Jerusalem (IL)**

(72) Inventor: **Ben-Sasson, Shmuel**
**10 Kiryat Moshe Street**
**Jerusalem (IL)**

**Eilat, Dan**
**20 Burla Street**
**Jerusalem (IL)**

(74) Representative: **Green, Alan James et al**
**SANDERSON & CO. European Patent Attorneys 34, East**
**Stockwell Street**
**Colchester Essex CO1 1ST (GB)**

(54) Pharmaceutical compositions containing polyaromatic compounds as active ingredient and their use.

(57) The present invention relates to pharmaceutical compositions containing polyaromatic compounds as active ingredient and their use. In the invention the pharmaceutical composition is one for affecting tissue redistribution of bioactive peptides and proteins which are normally bound to glycosaminoglycans. Also the composition is for mimicking the action of glycosaminoglycans in biological interactions. The composition comprises as active ingredient in combination with a pharmacologically acceptable carrier, a therapeutically effective amount of a pharmacologically acceptable aromatic compound. That compound is one containing between about 2 and about 10 aromatic rings, electronegative substituents on at least two of the aromatic rings and also containing negatively charged residues on at least two of the rings.

Bundesdruckerei Berlin

**Description**

## PHARMACEUTICAL COMPOSITIONS CONTAINING POLYAROMATIC COMPOUNDS AS ACTIVE INGREDIENT AND THEIR USE

The present invention relates to pharmaceutical compositions containing polyaromatic compounds as active ingredient therein.

More particularly the present invention relates to a pharmaceutical composition for affecting tissue redistribution of bioactive peptides and proteins normally bound to glycosaminoglycans and for mimicking the action of glycosaminoglycans in various biological interactions.

The term tissue redistribution as used herein implies either a change in the compartmentalization of a constituent within a given tissue or a change in the distribution pattern between different tissues. For example, the release of a peptide from a basement-membrane which can result in its consumption by surrounding cells or its transfer into the blood stream. Alternatively, it can be exemplified by a competitive binding of a soluble compound to a protein, thereby preventing its association with certain tissue-fixed residues.

As is known, glycosaminoglycans (= acid mucopolysaccharides) are a major constituent participating in the composition of various biological structures such as basement membranes, connective tissues, cartilage and the cell-surface glycocalyx.

Connective tissues are responsible for providing and maintaining form in the body. Functioning in a mechanical role, they provide a matrix that serves to connect and bind the cells and organs and ultimately give support to the body. Unlike the other tissue types (epithelium, muscle, and nerve) formed mainly by cells, the major constituent of connective tissue is its extracellular matrix, composed of protein fibers, an amorphous ground substance, and tissue fluid, the latter consisting primarily of bound water of solvation. Embedded within the extracellular matrix are the connective tissue cells.

In terms of structural composition, connective tissue can be subdivided into three classes of components: cells, fibers, and ground substance. The wide variety of connective tissue types in the body represents modulations in the degree of expression of these three components.

The amorphous intercellular ground substance fills the space between cells and fibers of the connective tissue; it is viscous and acts as a lubricant and also as a barrier to the penetration of the tissues by foreign particles. The ground substance is formed mainly by two classes of components: glycosaminoglycans and structural glycoproteins.

Glycosaminoglycans (GAG) are linear polysaccharides formed by characteristic repeating disaccharide units usually composed of a uronic acid and a hexosamine. The term acid mucopolysaccharides was used originally to designate hexosamine-rich acid polysaccharides extracted from connective tissue. In recent years, the term glycosaminoglycans has gained greater acceptance and is now used in place of mucopolysaccharides. The hexosamine can be glucosamine or galactosamine, and the uronic acid can be glucuronic or iduronic acid. Sulphate groups are found on all glycosaminoglycans apart from hyaluronic acid, and all of the sulphated glycosaminoglycans are covalently linked to protein forming different classes of proteoglycans. However, it would be an oversimplification to conceive of the glycosaminoglycans as simple repeat-unit polysaccharides, since considerable chemical and configurational variability can be superimposed upon the component sugars.

Among other functions it has been shown that the glycosaminoglycans serve also as a support which binds various bioactive peptides. This association is based on a non-covalent interaction since the bound protein can be readily released upon the addition of free glycosaminoglycans such as heparin. Well known examples include enzymes such as lipoprotein lipase (= LPL) or growth-regulating peptides such as fibroblast growth factor (FGF). In addition, it has been demonstrated that heparin inhibits the growth of vascular smooth muscle cells and the proliferation of kidney mesangial cells. The former cell type is involved in atherosclerosis while the latter plays a role in glomerulosclerosis. Another example of GAG-protein interaction is that of the enzyme heparanase which participates in cell-invasion processes.

In accordance with the present invention it has now been realized that the ability to compete with the binding of bioactive peptides to glycosaminoglycans, via the application of synthetic, biocompatible, molecules, opens the way for therapeutical manipulations of many important systems.

In accordance with the present invention there has now been found a family of synthetic low-molecular-weight compounds which can modulate biological systems containing complexes between bioactive peptides (proteins) and glycosaminoglycans and can mimic the action of glycosaminoglycans in various biological interactions.

These compounds have various pharmaceutical applications by causing a change in the tissue distribution of glycosaminoglycan-associated molecules which are involved in pathological or physiological processes. Thus pharmaceutical compositions can be prepared for:

1. Removal of cationic proteins from the glomerular basement membrane or connective-tissues, thereby preventing local damage (i.e. via "recharging" or negatively charged residues in the glomerular basement membrane).

2. Modulation of LPL, a key enzyme in lipid distribution among various tissues (= cardiovascular diseases).

3. Release of growth-promoting molecules, such as fibroblast growth factor (FGF), from basement membranes in order to enhance the process of angiogenesis and wound-healing. In addition, FGF can prevent death of lesioned neurons (see Anderson K.J. et al. Nature 332:360-1 (1988). Therefore, the release of endogenously-stored FGF might be beneficial for the treatment of neuronal disorders such a Alzheimer's disease and other dementia.

4. Blocking the activity of heparanase, an enzyme which participates in inflammatory processes and metastases formation.

5. Modulation of bone metabolism.

6. Control of the proliferation of certain cell types such as smooth muscle cells or mesangial cells.

These and other similar results can be achieved according to the present invention which provides a pharmaceutical composition for affecting tissue redistribution of bioactive peptides and proteins which are normally bound to glycosaminoglycans and for mimicking the action of glycosaminoglycans in biological interactions including heparin-like activity, comprising, as active ingredient in combination with a pharmacologically acceptable carrier, a therapeutically effective amount of a pharmacologically acceptable aromatic compound containing between about 2 and about 10 aromatic rings, electronegative substituents on at least two of the aromatic rings and also containing negatively charged residues on at least two of the rings.

As is known, heparin is a commercially available glycosaminoglycan useful in the release of lipoprotein lipase, the inhibition of heparanase or the release of fibroblast growth factor. The most common application of heparin is as an anticoagulant where this GAG molecule interacts with proteins which play a key role in hemostasis. Thus in another aspect of the present invention, pharmaceutical compositions of the present invention can be used as anticoagulants.

In preferred compounds used in the pharmaceutical compositions of the present invention, preferably the compounds contain aromatic domains comprising between two and ten aromatic rings; an electronegative substituent(s) ( = permanent dipole) on at least two of the aromatic ring structures (such as -NH$_2$, -NH-, -N = , -OH, = O or a halogen), and negatively charged residues (such as -COO$^-$; -O$^-$ or -SO$_3^-$) on at least two of the rings.

As will be seen in the examples hereinafter in the compounds used in the pharmaceutical composition of the present invention said negatively charged residue is preferably balanced by a pharmaceutically acceptable counter-ion. Thus said compound can be a salt with a cation such as sodium, potassium or ammonium or in compounds wherein said negatively charged residue is COO$^-$ said compound can be a C$_1$-C$_{12}$ ester thereof.

The preferred number of aromatic rings is between three and six.

While at least one of said rings can be condensed in a polycyclic structure, it is not preferred that all the rings be condensed and preferably at least two of said aromatic rings are phenyl rings.

The molecular weight of these compounds is preferably less than 1500 and even less than 1000.

Especially preferred compounds are derivatives of triphenylmethane such as aluminon, halogenated aluminon, sulfonated aluminon, sulfonated-halogenated aluminon and methyl blue.

Many known synthetic dyes having the above defined characterizing structural features can be used in the pharmaceutical compositions of the present invention. Several examples are listed in Table I alongside with their presently known therapeutical application. As can be seen, some of the compounds have been used merely as a color additive. Others were used as a diagnostic aid administered by an i.v. injection; while some of the rest were used in the past as antiseptic agents or as a remedy for infectious diseases. Nevertheless, none of these compounds has previously been described or used as a therapeutic agent which replace glycosaminoglycans.

EP 0 354 818 A2

## TABLE I

| Generic name[*] | Compound name[*] | Use |
|---|---|---|
| Aluminon | 5-[(3-Carboxy-4-hydroxyphenyl) (3-carboxy-4-oxo-2,5-cyclohexadien-1-ylidene)methyl]-2-hydroxybenzoic acid triammonium salt | Pharyngeal aerosol spray |
| Halogenated Aluminon | Halogen(s) substituted Aluminon (replacing one or more of the hydrogens on the aromatic rings) | – |
| Sulfonated Aluminon | Aluminon in which one or more of the carboxylic groups are replaced by sulfonate residue(s) | |
| Sulfonated Halogenated Aluminon | | |
| Anazolene Sodium | 4-Hydroxy-5-[[4-(phenylamino)-5-sulfo-1-naphthalenyl]azo]-2.7-naphthalenedisulfonic acid trisodium salt; | Diagnostic aid (plasma volume indicator) |
| Eosine I Bluish | 4',5'-Dibromo-3',6'-dihydroxy-2',7'-dinitrospiro[isobenzofuran-1(3H),9'-[9H]xanthen]-3-one disodium salt | Dye and Stain |
| Eosine yellowish | 2',4',5',7'-Tetrabromo-3',6'-dihydroxyspiro[isobenzofuran-1(3H) 9'-[9H]xanthen]-3-one disodium salt | FDA approved color additive for drugs |
| Erythrosine | 3',6'-Dihydroxy-2',4',5',7'-tetraiodospiro[isobenzofuran-1(3H), 9'-[9H]xanthen]-3-one disodium salt | FDA approved color additive for drugs |
| Evan's Blue | 6,6'-[(3,3'-Dimethyl[1,1'-biphenyl] | Diagnostic aid |

4

| | | |
|---|---|---|
| | 4,4'-diyl)bis(azo)]bis[4-amino-5-hydroxy-1,3-naphthalenedisulfonic acid] tetrasodium salt | (blood volume determin) |
| Fast Green FCF | N-Ethyl-N-[4-[[4-[ethyl[(3-sulfophenyl)methyl]amino]phenyl](4-hydroxy-2-sulfophenyl)-methylene]-2,5-cyclohexadien-1-ylidene]-3-sulfobenzene-methanaminium hydroxide inner salt, disodium salt; | FDA approved color additive for drugs |
| Fuchsin(e) Acid | 2-Amino-5-[(4-amino-3-sulfophenyl)(4-imino-3-sulfo-2,5-cyclohexadien-1-ylidene)methyl]-3-methylbenzenesulfonic acid dissodium salt; | pH indicator, biological stain |
| Iodophthalein Sodium | 3,3-Bis(4-hydroxy-3,5-diiodophenyl)-1(3H)-isobenzofuranone sodium salt; | Diagnostic aid radiopaque medium |
| Methyl Blue | [[4-Bis[4-(sulfophenyl)amino]-phenyl]methylene]-2,5-cyclohexadien-1-ylidene]amino]benzenesulfonic acid disodium salt; | Antiseptic |
| Rose Bengal | 4,5,6,7-Tetrachloro-3',6'-dihydroxy-2',4',5',7'-tetraiodospiro[isobenzofuran-1(3H),9'-[9H]xanthen]-3-one dipotassium salt; | Diagnostic aid (as radioactive $I^{131}$ derivative) |
| Sulfobromophthalein Sodium | 3,3'-(4,5,6,7-Tetrabromo-3-oxo-1(3H)-isobenzofuranylidene)bis[6-hydroxybenzenesulfonic acid] disodium salt; | |
| Suramin Sodium | 8,8'-[Carbonylbis[imino-3,1-phenylenecarbonylimino(4-methyl-3,1-phenylene)carbonylimino]]bis-1,3,5-naphthalenetrisulfonic acid hexasodium salt; | Antitripanosomal Antifilarial |
| Trypan Blue | 3,3'-[(3,3'-Dimethyl[1,1'-biphenyl]-4,4'-diyl)bis(azo)]bis[5-amino-4-hydroxy-2,7-naphthalenedisulfonic acid] tetrasodium salt; | —— |
| Trypan Red | 4,4'-[(3-Sulfo[1,1'-biphenyl]-4,4'- | Antitripana- |

5

diyl)bis(azo)]bis[3-amino-2,7-          somal
naphthalenedisulfonic acid]
pentasodium salt;         —

*  According to the Merck Index, 10th Ed. (1983)

The above Table I contains a list of compounds which can be used in the present invention, according to their description in the Merck Index. It is obvious, however, that the aromatic anions are the active component and therefore, all pharmaceutically acceptable salts of the listed compounds are intended to be included herein. For example, aluminon is an ammonium salt of aurintricarboxylic acid; however, the potassium or sodium salt or the free acid are obviously also included.

Likewise, aluminon, sulfonated aluminon, halogenated aluminon or sulfonated-halogenated aluminon, can be given orally as $C_1$-$C_{12}$ esters of the parental compounds in order to facilitate their intestinal absorption. Cellular esterases will then convert them back to aurintricarboxylic acid or its corresponding derivatives.

One of the compounds listed above, aluminon and certain of its derivatives and salts, were disclosed by Bernstein et al. (U.S. Patent 4,007,270) as inhibitors of the complement system of warm-blooded animals. This effect can be attributed to a non-specific enzyme inhibition by aluminon (see Bina-Stein M. and Tritton M., Molec. Pharmacol. 12, 191 (1976)), since the activation of the complement system involves several enzymatic steps.

Therefore said patent does not teach or suggest that aluminon or its derivatives can be used as therapeutic agent which mimics the action of glycosaminoglycans in biological interactions.

As will be realized complexes between proteins and glycosaminoglycans are involved in many different systems in the body. Consequently, the target tissue for the hereby proposed pharmaceutical compositions might vary between one patho-physiological condition to the other.

Therefore it will be realized that the present invention provides a new approach and a new family of compositions for treating different medical conditions although obviously different compounds will be selected for different conditions.

Thus, in the case of nephropathies where the target structure is the glomerular basement membrane (GBM) in the kidney, the compound of choice will be one which readily leaves the blood stream and is filtered through the GBM. When the target is a component circulating in the blood stream (e.g. antithrombin III or is a complex located on the surface of endothelial cells which line blood vessels, as in the case of LPL, the compound will be one that preferably will be retained in the vascular compartment, (e.g., via its attachment to the serum albumin). Yet another example is the release of growth factor(s) to assist the process of wound-healing. In such a condition it is advantageous to maintain the drug locally, i.e., to use compounds which become firmly bound to connective tissue constituents and do not diffuse away.

In addition, the release of endogenously-stored growth factor(s) might be beneficial for the treatment of neuronal disorders. In such a case it is advantageous to select compounds which are able to cross the blood-brain barrier.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

## EXAMPLE 1

Modulation of lipoprotein lipase (LPL) levels and compartmentalization

The enzyme LPL participates in the process of lipid transfer from the bloodstream to the tissues, LPL is bound to the external surface of cells via its association with cell-membrane glycosaminoglycans. Cultured heart cells is a powerful system in the study of the enzyme turn-over and the exploration of ways to manipulate its level since these cells are active in the biosynthesis of LPL, in addition to the surface binding of the enzyme.

The following are the material and methods:

$F_1$ heart cell cultures were prepared from newborn rat hearts as previously described (Chajek, T. et al, Biochem. Biophys. Acta 528, 456-474(1978)). They consisted mainly of non-beating mesenchymal cells and were used 8-12 days after their isolation.

The enzyme activity was determined on aliquots of medium and of homogenates of cells which had been released from the petri dish with a rubber policeman in 1 ml of 0.025 M $NH_3$/$NH_4Cl$ buffer (pH 8.1) containing 1 unit/ml of heparin. The assay system consisted of 0.1 ml of medium or 0.1 ml of cell homogenate (50-70 g protein) and 0.1 ml of substrate containing labeled triolein, prepared according to the method of Nilsson-Ehle and Schotz, (J. Lipid Res. 17, 536-541 (1976)). Incubations were carried out at 37°C for 45 min. The reaction was stopped by the addition of methanol/chloroform/heptane (1,4:1.25:1, v/v) and the extraction of fatty acids was performed according to the method of Belfrage and Vaughan (J. Lipid Res. 10, 341-344 (1969), as modified by Nilsson-Ehle and Schotz. Enzyme activity was calculated according to the formula of Nilsson-Ehle and Schotz.

Table II demonstrates the ability to modulate (shift-up) the enzyme level by Evan's Blue and to influence its compartmentalization. Similar results were obtained with Methyl Blue, Trypan Blue and Aluminon.

## TABLE II.

### LPL activity following 24 hrs incubation of heart cells with Evan's Blue

| Evans's Blue concentration (M) | LPL activity: m mole FFA/h/dish (mean±SEM of triplicates) | | | % increase in the total |
|---|---|---|---|---|
| | cells | medium | total | |
| 0 | 2420±93 | 817±14 | 3237 | -(0) |
| 10⁻⁵ | 2757±114 | 1531±182 | 4288 | +32.5 |
| 10⁻⁴ | 2447±48 | 2783±194 | 5230 | +61.6 |
| 10⁻³ | 1698±184 | 6051±435 | 7749 | +139.4 |

## EXAMPLE 2:

Inhibition of heparanase activity

Heparanase is an endoglucuronidase capable of degrading heparan sulfate (HS) at specific intrachain sites. Studies on degradation of sulfated proteoglycans in the subendothelial extracellular matrix (ECM) demonstrated a correlation between heparanase activity and the metastatic potential of various tumor cells. Heparanse activity was also suggested to play a role in the mobilization of normal circulating cells of the immune system during inflammatory processes.

The ability of several of the above mentioned compounds to inhibit lymphoma-cell derived heparanase was tested in the assay system described by Vlodavsky et. al. (Cancer Research. 43: 2704-2711 (1983). ³⁵S labelled ECM was incubated for 24 hrs with ESb mouse lymphoma heparanase in the presence of 10 ugr ml of the indicated compounds. Degradation of the sulfated glycosaminoglycan was followed by gel filtration of the supernatants. Heparanase activity is expressed as the total amount of labelled low-molecular-weight fragments released from the ECM substrate. The results are summarized in Table III.

## TABLE III

### Inhibition of heparanase activity by various compounds

| Compound (10 μgr/ml) | Total low-mol-wt. HS fragments released by heparanase (cpm) | % Inhibition |
|---|---|---|
| Control | 17,756 | - |
| Methyl Blue | 13,676 | 23 |
| Fast Green | 12,139 | 32 |
| Evan's Blue | 6,986 | 60 |
| Aluminon | 3,822 | 78 |

## EXAMPLE 3:

Anticoagulant effect

The anticoagulant effect of aluminon was demonstrated by measuring the partial thromboplastin time (PTT) in blood samples from a normal donor. 0.25 ml of the indicated concentration of the drug (x10, diluted in saline) were added to 2.25 ml of fresh human blood, collected in the presence of citrate. PTT was measured following the addition of Ca⁺⁺. The results are summarized in the following Table IV.

TABLE IV

PTT of human blood in the presence of various
concentrations of aluminon

| Aluminon final conc. (mg/ml) | PTT (sec.) |
|---|---|
| 1 | >300 |
| 0.1 | 190 |
| 0.01 | 30.5 |
| 0 (control) | 30.5 |

As can be seen, aluminon is an effective anticoagulant. Moreover, since aluminon can be absorbed through the gastrointestinal tract, it can be used as an orally administrable anticoagulant (as opposed to heparin which has to be given by injection). Indeed, an oral administration of aluminon to rats (1ml of 10%-20% solution) prolonged their PTT considerably (from around 20 sec. at time 0 to >200 sec. after 2-4 hours); concomitantly, plasma levels of LPL were elevated by about an order of magnitude.

## EXAMPLE 4:

Inhibition of the proliferation of vascular-smooth muscle cells.

Bovine aortal smooth-muscle cells were grown in tissue culture plates in the presence of glucose-rich medium (H-21). The cultures (around $35 \times 10^3$ cells/well in 24 wells' plates) were initially supplemented with 10% fetal calf serum (FCS) for 2-3 days. The cell nutrition was then replaced by a serum-deprived medium (H-21 plus 0.2% FCS) in order to test the effect of defined growth factors on the proliferation of smooth-muscle cells and their possible inhibition by the compounds with heparin-like activity.

In the following representative experiment, the cells transferred to the serum-deprived medium were grown in the presence of thrombin ($10^{-6}$M) or FGF (partially purified from bovine brain, 250 ngr/ml). Various concentrations of aluminon were added to the thrombin or FGF-treated cells and to non-treated control cultures. $^3$H thymidine was also added to all wells for an incubation period of 2 days. The cultures were then harvested and the $^3$H thymidine incorporated into DNA was measured. The results are shown in Table 5.

TABLE V

Growth inhibition of resting, FGF or
thrombin-stimulated smooth muscle cells by
aluminon

| Aluminon Concentration (µgr/ml) | $H^3$ thymidine incorporation per well (cpm, mean of duplicates) | | |
|---|---|---|---|
| | Control | +FGF | +Thrombin |
| 0 | 9,823 | 22,574 | 54,000 |
| 0.2 | 8,425 | 22,256 | 46,857 |
| 2 | 5,120 | 13,089 | 14,064 |
| 10 | 4,105 | 6,846 | 6,877 |
| 20 | 4,343 | 6,745 | 5,705 |

More than 40% inhibition is already achieved at aluminon concentration of 2 µgr/ml ($\sim 4 \times 10^{-6}$M).

In other words, the compound can mimic the action of heparin also in this system as a potent inhibitor of the proliferation of vascular smooth muscle cells.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A pharmaceutical composition for affecting tissue redistribution of bioactive peptides and proteins which are normally bound to glycosaminoglycans, and for mimicking the action of glycosaminoglycans in biological interactions, comprising, as active ingredient in combination with a pharmacologically acceptable carrier, a therapeutically effective amount of a pharmacologically acceptable aromatic compound containing between about 2 and about 10 aromatic rings, electronegative substituents on at least two of the aromatic rings and also containing negatively charged residues on at least two of the rings.

2. A pharmaceutical composition according to claim 1 wherein said electronegative substituents are selected from $-HN_2$, $-NH-$, $-N=$, $=OH$, $=O$ or halogen.

3. A pharmaceutical composition according to claim 1 or claim 2 wherein said negatively charged residue is selected from $-COO^-$, $-O^-$ or $-SO_3^-$.

4. A pharmaceutical composition according to any one of the preceding claims wherein said negatively charged residue is balanced by a pharmaceutically acceptable counter cation.

5. A pharmaceutical composition according to claim 4 wherein said compound is a salt.

6. A pharmaceutical composition according to claim 5 wherein said salt contains sodium, potassium or amonium as the balancing cation.

7. A pharmaceutical composition according to any one of claims 1 to 3 wherein said negatively charged residue is $COO^-$ and said compound is a $C_1$-$C_{12}$ ester thereof.

8. A pharmaceutical composition according to any one of the preceding claims having anticoagulant properties.

9. A pharmaceutical composition according to any one of the preceding claims wherein said compound contains between 3 and 6 aromatic rings.

10. A pharmaceutical composition according to any one of the preceding claims wherein at least two of said rings are phenyl rings.

11. A pharmaceutical composition according to any one of claims 1 to 9 wherein at least one of said rings is condensed in a polycyclic structure.

12. A pharmaceutical composition according to any one of the preceding claims wherein said compound has a molecular weight of less than 1500.

13. A pharmaceutical composition according to claim 12 wherein said compound has a molecular weight of less than 1000.

14. A pharmaceutical composition according to any one of the preceding claims wherein said compound is a derivative of triphenylmethane.

15. A pharmaceutical composition according to claim 1 wherein said compound is selected from Aluminon, Halogenated Aluminon, Sulfonated Aluminon, Sulfonated-Halogenated Aluminon, Anazolene Sodium, Eosine I Bluish, Eosine Yellowish, Erythrosine, Evan's Blue, Fast Green FCF, Fuchsine Acid, Iodophthalein Sodium Methyl Blue, Rose Bengal, Suramin Sodium, Trypan Blue and Trypan Red.

16. The use of a therapeutically effective amount of a pharmacologically acceptable aromatic compound containing between about 2 and about 10 aromatic rings, and having electronegative substituents on at least two of the aromatic rings and negatively charged residues on at least two of the rings, for the manufacture of a medicament for affecting tissue redistribution of bioactive peptides and proteins normally bound to glycosaminoglycans, and for mimicking the action of glycosaminoglycans in biological interactions.

17. As active ingredient in combination with a pharmacologically acceptable carrier, a therapeutically effective amount of a pharmacologically acceptable aromatic compound containing between about 2 and about 10 aromatic rings having electronegative substituents on at least two of the aromatic rings and negatively charged residues on at least two of the rings for affecting tissue redistribution of bioactive peptides and proteins which are normally bound to glycosoaminoglycans and for mimicking the action of glycosaminoglycans in biological interactions.